# EUROPEAN PATENT SPECIFICATION

(11) **EP 3 639 857 B1**
(45) Date of publication and mention of the grant of the patent: **31.07.2024**
(21) Application number: 18818459.2
(22) Date of filing: 11.06.2018
(51) Int. Cl.: A61K 47/60, A61K 38/18, C07K 14/505

(54) **ERYTHROPOIETIN COMPOSITION WITH IMPROVED STABILITY AND METHOD FOR PREPARING SAME**
ERYTHROPOIETINZUSAMMENSETZUNG MIT VERBESSERTER STABILITÄT UND HERSTELLUNGSVERFAHREN DAFÜR
COMPOSITION D'ÉRYTHROPOÏÉTINE À STABILITÉ AMÉLIORÉE ET PROCÉDÉ DE PRÉPARATION DE CETTE DERNIÈRE

(30) Priority: 12.06.2017 KR 20170073111
(43) Date of publication of application: 22.04.2020
(73) Proprietor: Hankook Korus Pharmaceutical Co. Ltd., Chuncheon-si, Gangwon-do 24398 (KR)
(72) Inventor: WHANG, Jae Gan, Seoul 07703 (KR); HAN, Sang In, Incheon 21982 (KR); CHO, Jeong Haeng, Gimpo-si Gyeonggi-do 10091 (KR); KWON, Sung Hun, Incheon 21120 (KR); KANG, Min Ji, Seoul 07282 (KR); JUNG, Bong Jun, Seoul 07294 (KR)
(74) Representative: Ström & Gulliksson AB
(86) International application number: PCT/KR2018/006593
(87) International publication number: WO 2018/230903

(56) References cited:
- WO-A1-2009/079911
- WO-A2-01/76640
- WO-A2-2005/092369
- WO-A2-2007/104768
- KR-A- 20060 032 140
- US-B2- 8 765 924
- EMMA M. PELEGRI-O'DAY ET AL: "Therapeutic Protein-Polymer Conjugates: Advancing Beyond PEGylation", JOURNAL OF THE AMERICAN CHEMICAL SOCIETY, vol. 136, no. 41, 15 October 2014 (2014-10-15), US, pages 14323 - 14332, XP055409454, ISSN: 0002-7863, DOI: 10.1021/ja504390x
- MACDOUGALL I C ET AL: "Novel strategies for stimulating erythropoiesis and potential new treatments for anaemia", THE LANCET, ELSEVIER, AMSTERDAM, NL, vol. 368, no. 9539, 9 September 2006 (2006-09-09), pages 947 - 953, XP027947014, ISSN: 0140-6736, [retrieved on 20060909]
- HANIU M ET AL: "Recombinant human erythropoietin (rHuEPO): cross-linking with disuccinimidyl esters and identification of the interfacing domains in EPO", PROTEIN SCIENCE, WILEY, US, vol. 2, no. 9, 1 September 1993 (1993-09-01), pages 1441 - 1451, XP002427244, ISSN: 0961-8368
- COHAN, R. A. ET AL.: "Design, Modeling, Expression, and Chemoselective PEGylation of a New Nanosize Cysteine Analog of Erythropoietin", INTERNATIONAL JOURNAL OF NANOMEDICINE, vol. 6, 2011, pages 1217 - 1227, XP055566570

## Description

### Field of the Invention

The present invention relates to a method of preparing an erythropoietin conjugate with improved stability and to the resulting conjugate as well as to a pharmacological composition comprising the conjugate as an active ingredient.

### BACKGROUND OF THE INVENTION

### Description of the Related Art

Erythropoietin (EPO) is a hormone that regulates the final maturation and growth of erythrocyte progenitor cells and is mainly produced in fetal liver or adult kidney. It is known that the increase of an amount thereof in the blood in a hypoxic blood state regulates the production of mature oxygen transferring erythrocyte cells. If the number of erythrocytes decreases due to the decrease of EPO in the blood, tissue dysfunction occurs, which leads to anemia. Therefore, in recent years, a technology of administering EPO, which is artificially synthesized in vitro, as a pharmaceutical composition has been developed. Because biopharmaceuticals such as polypeptides like EPO, aptamers, and siRNAs generally have low stability, they are easily denatured and degraded by proteolytic enzymes in the blood to be easily removed through the kidneys or liver. Therefore, protein drugs need to be more frequently administered to patients in order to maintain blood levels and titers of protein medicines including polypeptide as pharmacological ingredients. However, in the case of protein medicines, which are mostly administered to patients in the form of injections, frequent injections for the maintenance of the blood level of active polypeptides not only cause pain for the patient but also require high cost for storage and distribution of the manufactured protein medicines. Accordingly, there are urgent demands for technologies for reducing the decrease of physiological activity of biopharmaceuticals as much as possible and increasing blood half-life.

Haniu M et al (Protein Science (1993), 2, 1331-1451 discloses cross-linking of recombinant human erythropoietin (rHuEPO) with disuccinimidyl esters and identification of the interfacing domains in EPO.

KR 2006 0032140 A discloses biologically active erythropoietin (EPO) conjugate compositions wherein a transglutaminase reaction is employed to covalently and site specifically conjugate the EPO molecule to a non- antigenic hydrophilic polymer that can also be covalently linked to an organic molecule either of which modification increases the circulating serum half-life of the composition.

WO 2007/104768 discloses di-polymer protein conjugates, such as di-polymer conjugates of erythropoietin, interferon and human growth hormone, as well as processes for their preparation.

Meanwhile, PEGylation (covalent conjugation) is a DDS (drug delivery system) technology for maximizing the plasma half-life of existing protein drugs, continuously maintaining medical effect thereof in vivo, and reducing immunogenicity and antigenicity, which are side effects of existing drugs, caused by the frequent administration. PEGylation is a water-soluble biocompatible polymer covalently binding to a protein/peptide and has a function of changing physical properties of the protein to increase body stability. PEGylation sustaining preparations of protein medicines should increase the stability of the protein medicines while simultaneously maintaining a sufficiently high titer of the drug itself and should not cause an immune response in the patient. However, most of the PEGylation is non-selectively performed, and thus there is a problem such as blocking due to the PEGylation of a site showing the biological activity, which causes problems of significantly reducing the activity or showing nonuniformity in activity for each PEGylation site.

US 8 765 924 B2 relates to pegylated proteins, such as pegylated-erythropoietin, and their methods of making and use.

As described above, the inventors of the present invention have continuously conducted research for compensating the disadvantages of the conventional PEGylation technology and developing a method for producing EPO conjugated with a biocompatible polymer having high reaction yield and high bio-yield and having high body stability and high sustainability to complete the present invention. The erythropoietin composition with improved stability according to the present invention has prominently increased body stability, and thus is expected to be greatly utilized in the improvement on the medical field and the medical welfare of human beings.

### SUMMARY OF THE INVENTION

An object of the present invention is to provide an erythropoietin composition with improved stability and a method for preparing the same.

Hereinafter, various embodiments described herein are described with reference to the drawings. In the following description, for thorough understanding of the present invention, various specific details, for example, specific forms, compositions, processes, and the like are described. However, specific embodiments may be practiced without one or more of these specific details, or in conjunction with other known methods and forms. In other instances, known processes and manufacturing techniques are not described in particular detail in order to not unnecessarily obscure the present invention. Reference throughout this specification to "one embodiment" or "embodiment" means that particular features, forms, compositions, or characteristics described in connection with the embodiment are included in one or more embodiments of the invention. Therefore, the context of "in one embodiment" or "embodiment" expressed at various places throughout this specification does not necessarily represent the same embodiment of the present invention. In addition, particular features, forms, compositions, or characteristics may be combined in any suitable manner in one or more embodiments.

"Erythropoietin (EPO)" is a glycoprotein hormone that has a function of controlling the final maturation and growth of erythroid progenitor cells (Goldwasser et al., Ann. NY Acad. Sci., 149, 49, 1968) and is mainly produced in fetal liver or adult kidney. The amount of human EPO in blood increases in a hypoxic blood state and as a result, the production of mature oxygen transferring erythrocyte cells is regulated. Because humans maintain a proper level of erythrocytes for normal tissue function, if the number of erythrocytes decreases, tissue dysfunction occurs to cause anemia. Human EPO was first known when the possibility of humoral factors capable of controlling erythropoiesis was reported in 1906, and it was found that a hormone that promotes the production of erythrocyte, called erythropoietin, was produced in the kidney in 1957 (Jacobson et al., Nature, 179, 633, 1957). After Miyake et al. succeeded in mass separation and purification from urine in patients with aplastic anemia in 1977 (Miyake et al., J. Biol. Chem., 252 (15), 5558, 1977), subsequent research in molecular biology and glycobiology has been accelerated. N-terminal 30 amino acid sequences were reported for the first time by Yanagawa et al. (Yanagawa et al., J. Biol. Chem., 295(5), 2707, 1984), and two years later, Lai et al. determined and reported the total amino acid sequence from EPO derived from human urine (Lai et al., J. Biol. Chem., 261, 3116, 1986). Natural human EPO is an acidic, monomeric glycoprotein, a total molecular weight thereof is about 34,000 Daltons, and a molecular weight of only the protein portion excluding sugars is about 18,000 Daltons (Wang et al., Endocrinology, 116, 2286, 1985). It is known that, a sugar chain portion which takes up about 40% of the total molecular weight in human EPO has an important function in the in vivo activity and structural stability of human EPO (Linda et al, J. Biological Chemistry 266, 23022-23026 (1991)). Therefore, when the sugar content of human EPO is reduced, the plasma half-life of the human EPO molecule is drastically reduced and the biological activity of human EPO is lost (Goto et al., Biotechnology, 6, 67, 1988).

In recent years, a technique for administering EPO, that is artificially synthesized in vitro, as a pharmaceutical composition has been developed. The EPO was prepared using recombinant DNA technology through gene cloning and expression and has been usefully used in treating various forms of anemia including the anemia associated with renal failure and cancer patients in myelosuppression chemotherapy (Lin, US5618698). However, although the efficacy of such EPO has been demonstrated, EPO has a short half-life in the body (approximately 4 hours) and should be frequently administered, and thus there is a limit to satisfying the patients. Thus, research on the improvement of the stability and sustainability in the body while maintaining the intrinsic activity of commercially available protein remedies such as EPO is continuously performed.

In the present disclosure, "PEGylation" means "covalent attachment" and is a DDS (drug delivery system) technology for maximizing the plasma half-life of existing protein drugs, continuously maintaining medical effect thereof in vivo, and reducing immunogenicity and antigenicity, which are side effects of existing drugs caused by the frequent administration. PEGylation is a water-soluble biocompatible polymer covalently binding to a protein/peptide and has a function of changing physical properties of the protein to increase body stability. However, most of the PEGylation is non-selectively performed, and thus there is a problem such as blocking due to the PEGylation of a site showing the biological activity, which causes problems of significantly reducing the activity or showing nonuniformity in activity for each PEGylation site.

Until recently, eight pegylated products have been used as remedies, and in particular, PEGylation-interferon, a hepatitis C treatment approved by the US FDA in 2002, was developed as once-weekly injections, market share thereof exceeded 1.3 billion dollars a year, and more than 70 percent of the market of first-generation interferon, which is three times per week injections, is rapidly replaced.

In the present invention, the PEGylation is performed on the EPO. It is most preferred to be PEGylated with polyethylene glycol (PEG).

In the present disclosure, a "biocompatible polymer" collectively refers to all biocompatible polymers useful for the human body which can be added to biologically active materials such as EPO, such as natural or artificial synthetic polymer materials. The expression "biocompatibility" used in the present specification refers to the ability of not causing a toxic response, an inflammatory response, an immune response, carcinogenicity, or the like, being non-toxic and harmless to a living body, not causing immunological rejections, and being compatible with body tissue or body system with good affinity.

In the present invention, the biocompatible polymer is polyethylene glycol. PEG is a water-soluble and non-toxic polymer with a repeating structure of HO-(-CH₂CH₂O-)-H. It is known that PEG has an effect of increasing the plasma half-life, increasing solubility and stability, and reducing immunogenicity. The molecular weight range of PEG bound to biologically active substances such as peptides or proteins is approximately 1,000 to 100,000 and preferably 5,000 to 40,000. It is known that, if the molecular weight of PEG is more than 1,000, the toxicity is quite low. PEG in the molecular weight range of 1,000 to 6,000 is distributed throughout the entire body and metabolized through the kidneys, and in particular, branched PEG having a molecular weight of 40,000 is distributed in organs including blood and liver and metabolized through the liver.

In order to bind a biocompatible polymer such as PEG to a biologically active substance such as protein, generally, one or both ends of the biocompatible polymer are substituted with a highly reactive functional group such as an aldehyde group, a succinimidyl group, a hydrozide group, or an imine group, and then the highly reactive functional group is bonded to an amine group, a carboxyl group, a thiol group, or the like of the biologically active substance through a chemical reaction to form a conjugate. In some cases, preferably, one hydroxy terminal of a biocompatible polymer such as PEG is protected with a methoxy group or the like, and then only the other terminal is reacted by the substitution with the highly reactive functional group, or one terminal is protected and a specific biologically active substance is bound to the other terminal, and then the protecting group at the other terminal is removed and activated such that another biologically active substance can be bound thereto.

As described above, a process of converting one or both of the terminal groups to the reactive functional group in order to bind the biocompatible polymer to the biologically active substance is referred to as "activation", and the polymer product produced by the substitution with the reactive functional group in this process is called "activated biocompatible polymers". For example, in order to bind poly(alkylene oxide) to peptide or protein, one of the hydroxyl terminal groups can be converted to a reactive functional group such as carbonate, and the resulting product becomes activated poly(alkylene oxide) (PAO) that is water-soluble at room temperature. Such groups include monosubstituted polyalkylene oxide derivatives such as mPEG or other suitable alkyl-substituted PAO derivatives such as those containing C1-4 terminal groups.

The reactive functional group that is used in the present invention is a group that activates the biocompatible polymer so as to bind to a carboxyl group of EPO.

"Polyethylene glycol (PEG)" is a kind of biocompatible polymer material, and many patent publications and documents for PEGylating a biologically active substance with PEG can be found. As an example of conjugating PEG derivatives to EPO, WO94/28024 discloses conjugation of PEG to an amine group of lysine of EPO or an oxidized group of sugar. US6340742B1 shows that the conjugation of 20K to 40K PEG derivatives to an amino group such as lysine on the surface of EPO increases half-life compared to EPO without PEG. Also, US6586398B1 shows erythrocyte production efficacy by conjugating a PEG derivative of linear or branched form to a free amine group or oxidized sugar chain containing an N-terminal of the novel erythropoietin stimulating protein (NESP) .

However, the existing conjugate of PEG-EPO is mainly obtained by conjugating PEG to an amine group of EPO, and there has been reported that, in case of many biologically active substances, for example, protein, if PEG is conjugated to lysine, significant biological activity is lost. It is reported that, EPO has only eight lysine groups, which may cause a problem on the reduction of activity, and as the number of PEG conjugated to lysine increases, the physiological activity of EPO is further reduced (WO94/28024). For example, in PCT/JP01/08539, it is reported that, if a PEG derivative is bound to an amine group of lysine of EPO, as the number of PEG bonds increases, only 13% to 0.79% of biological activity is maintained compared to native EPO.

Accordingly, there are urgent demands for the development of a PEG-EPO preparation method in which PEGylation is performed to EPO (PEG-EPO) to improve the stability of the EPO, while the titer of the biological activity is maintained.

In a first aspect of the present invention, there is provided a method of preparing a conjugate in which a compound represented by the formula (1) is conjugated to Lys 45 and 97 amino acids of erythropoietin, the method comprising: (a) a step of dissolving the compound represented by the formula (1) in phosphoric acid salt; (b) a step of adding erythropoietin in a molar ratio of 1:1 to 1:5 with respect to the compound represented by the formula (1) to (a) and stirring at 18° C to 32° C for 0.2 to 3 hours; and (c) a step of stopping reaction by adjusting acidity (pH) to 4.0 to 5.0

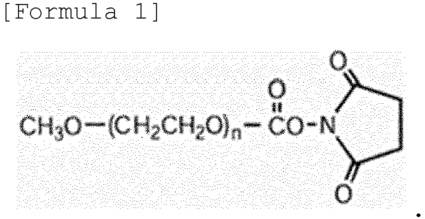

In a second aspect of the present invention, there is provided a conjugate obtainable by the method according to the present invention.

In a third aspect of the present invention, there is provided a pharmaceutical composition for use in preventing or treating a hematopoietic disease, wherein the pharmaceutical composition comprises the conjugate according to the present invention, as an active ingredient.

Herein, a "composition" refers to a polymer compound or a conjugate, which is present in a variety of similar structures. It includes same structural compounds, which are isomers.

The "hematopoietic disease" may be a disease in which hematopoiesis, that is, the production of blood cells is not normally regulated, and is an erythrocytic abnormality, a leukocyte abnormality, a hemorrhagic disease, and a lymphatic disease. Erythrocytic abnormality mainly refers to anemia, which is caused by decreased erythrocyte production, a hemolytic disease, and blood loss, and can be classified into iron-deficient anemia, pernicious anemia, aplastic anemia, and hemolytic anemia. Leukocyte abnormalities include leukemia and myeloma, and the leukemia is classified into lymphoid leukemia and myeloid leukemia, and the myeloma is classified into multiple myeloma and myeloid fibrosis. A hemorrhagic disease can be classified into a thrombocyte abnormality and a coagulation mechanism abnormality, the thrombocyte abnormality includes idiopathic thrombocytopenic purpura and thrombotic thrombocytopenia, and the coagulation mechanism abnormality include hemophilia, a von Willebrand disease, and a disseminated intravascular coagulation syndrome. In addition, the lymphatic disease is classified into inflammatory diseases of lymph nodes, malignant tumors, spleen diseases, and thymic diseases, and the inflammatory diseases of the lymph nodes can be classified into nonspecific lymphadenitis, tuberculous lymphadenitis and sarcoidosis, and necrotizing lymphadenitis.

Thus, according to one embodiment of the present invention, the hematopoietic disease is any one or more selected from the group consisting of iron deficiency anemia, pernicious anemia, aplastic anemia, hemolytic anemia, lymphoid leukemia, myeloid leukemia, multiple myeloma, myeloid fibrosis, idiopathic thrombocytopenic purpura, thrombotic thrombocytopenia, hemophilia, a von Willebrand disease, a disseminated intravascular coagulation syndrome, nonspecific lymphadenitis, tuberculous lymphadenitis & sarcoidosis, necrotizing lymphadenitis, lymphoid malignancy, spleen disease, and thymic disease.

"Administration" means introducing the composition of the present invention to the patient in any suitable method, and the route of administration of the composition of the present invention can be administered in any general route as long as the composition can reach a target tissue. Oral administration, intraperitoneal administration, intravenous administration, intramuscular administration, subcutaneous administration, intradermal administration, intranasal administration, pulmonary administration, rectal administration, intranasal administration, intraperitoneal administration, and intradural administration can be performed. The treatment method can include administering the pharmaceutical composition in a pharmaceutically effective amount. The effective amount can be regulated by various factors including the type of disease, the severity of a disease, types and amounts of active ingredients and other ingredients contained in the composition, the type of formulation, an age, a body weight, a general health condition, sex, and a diet of a patient, time of administration, a route of administration, a secretion rate of the composition, the duration of treatment, and the drugs used concurrently. In case of adults, when an expression inhibitor of gene or an activity inhibitor of protein is administered once or several times a day, the administration can be performed in the volume of 0.01 ng/kg to 10 mg/kg in case of siRNA, 0.01 ng/kg to 10 mg/kg in case of the antisense oligonucleotide for mRNA of the gene, 0.1 ng/kg to 10 mg/kg in case of a compound, and 0.1 ng/kg to 10 mg/kg in case of a monoclonal antibody to the protein.

Typically, the composition includes 0.001% to 100% by weight of the active ingredient relative to the total weight. The composition can further include a suitable carrier, an excipient, or a diluent according to conventional methods. Examples of the suitable carrier, excipient, or diluents that can be included in the composition of the present invention include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. The compositions can be respectively formulated in the form of oral formulations such as powders, granules, tablets, capsules, suspensions, emulsions, syrups, and aerosols, external preparations, suppositories, or sterile injectable solutions, according to conventional methods, to be used. Specifically, in case of being formulated, the preparation can be performed by using a diluent or an excipient such as a filler, an extender, a binder, a wetting agent, a disintegrating agent, and a surfactant, which are commonly used. Solid preparations for oral administration include tablets, pills, powders, granules, capsules, and the like, and such solid preparations can be prepared by mixing polymer polysaccharide of Chemical Formula 1 above or taro extract with at least one excipient, for example, starch, calcium carbonate, sucrose, lactose, gelatin and the like. In addition to simple excipients, lubricants such as magnesium stearate, and talc can also be used. Liquid preparations for oral use include suspensions, solvents, emulsions, and syrups, and in addition to water and liquid paraffin which are commonly used simple diluents, various excipients, for example, a wetting agent, a sweetening agent, fragrance, and a preservative can be included. Formulations for parenteral administration include a sterile aqueous solution, a non-aqueous solvent, a suspension, an emulsion, a lyophilized preparation, and a suppository. As a non-aqueous solvent and a suspending agent, propylene glycol, polyethylene glycol, vegetable oils such as olive oil, injectable esters such as ethyl oleate, and the like can be used. As the base of the suppository, witepsol, macrogol, TWEEN 61, cacao butter, laurin butter, glycerogelatin, and the like can be used.

The present disclosure provides a method of treating various medical diseases in a mammal, preferably a human. The method includes administering an effective amount of a PEG-EPO conjugate containing composition prepared as described herein to a mammal in need of such treatment. Among others, this conjugate is known in the medical field for the effects of hematopoiesis, and is useful for treating conditions that respond positively or optimistically to diseases based on hematopoietic dysfunction. Conditions that can be treated by a conjugate according to the present invention are generally diseases sensitive to a treatment using EPO. For example, the term "sensitive disease" is known in the medical arts, and the sensitive disease includes diseases which respond positively or optimistically to diseases based on hematopoietic dysfunction.

The present disclosure relates to a conjugate in which a biocompatible polymer compound is conjugated to erythropoietin, in which the biocompatible polymer compound is polyethylene glycol, wherein the polyethylene glycol is represented by Chemical Formula (1), and wherein the biocompatible polymer compound is conjugated to Lys 45 and 97 amino acids of the erythropoietin

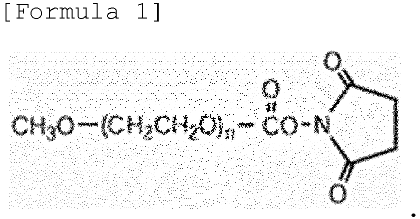

The present disclosure also relates to a pharmaceutical composition for use in preventing or treating a hematopoietic disease including the above conjugate as an active ingredient, and the hematopoietic disease is any one or more selected from the group consisting of iron-deficient anemia, pernicious anemia, aplastic anemia, hemolytic anemia, lymphoid leukemia, myeloid leukemia, multiple myeloma, myeloid fibrosis, idiopathic thrombocytopenic purpura, thrombotic thrombocytopenia, hemophilia, a von Willebrand disease, a disseminated intravascular coagulation syndrome, nonspecific lymphadenitis, tuberculous lymphadenitis and sarcoidosis, necrotizing lymphadenitis, lymphoid malignancy, spleen disease, and thymic disease.

Herein is also disclosed a method for preventing or treating a hematopoietic disease in a subject with the hematopoietic disease, comprising a step of administering an effective amount of any one or more of the conjugates for preventing or treating hematopoietic disease to a subject.

The present invention also provides a method of preparing a conjugate in which a compound represented by the formula (1) is conjugated to Lys 45 and 97 amino acids of erythropoietin, the method comprising: (a) a step of dissolving the compound represented by the formula (1) in phosphoric acid salt; (b) a step of adding erythropoietin in a molar ratio of 1:1 to 1:5 with respect to the compound represented by the formula (1) to (a) and stirring at 18° C to 32° C for 0.2 to 3 hours; and (c) a step of stopping reaction by adjusting acidity (pH) to 4.0 to 5.0 [Chemical Formula 1]

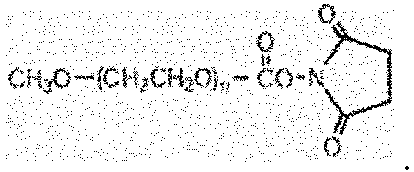

Hereinafter, the present invention is described in detail step by step.

### ADVANTAGEOUS EFFECT

The present invention provides a PEG-EPO conjugate, in which stability of erythropoietin (EPO) is improved and the titer of the biological activity is maintained, a composition including the PEG-EPO conjugate, and a method for preparing the PEG-EPO conjugate, and provides a therapeutic effect useful to diseases based on hematopoietic dysfunction, and thus it is expected to be greatly utilized for the improvement on the medical field and the medical welfare of human beings.

### BRIEF DESCRIPTION OF DRAWINGS

FIG. 1 is a diagram showing results of SDS PAGE analysis of PEG-EPO-TS according to an embodiment of the present invention.
FIG. 2 is a diagram showing results of performing HPLC purity analysis of PEG-EPO-TS according to an embodiment of the present invention.
FIG. 3 is a diagram showing results of SDS-PAGE separation and band staining of PEG-EPO-Mircera, PEG-EPO-TS, and EPO according to an embodiment of the present invention.
FIG. 4 is a diagram showing results of HPLC analysis of SDS-PAGE separated PEG-EPO-TS conjugate band according to an embodiment of the present invention.
FIG. 5 is a diagram showing results of producing PEG-EPO conjugates depending on pH reaction conditions according to an embodiment of the present invention.
FIG. 6 is a diagram showing results of producing PEG-EPO conjugates depending on EPO:PEG ratios according to an embodiment of the present invention.
FIG. 7 is a diagram showing results of producing PEG-EPO conjugates depending on reaction time according to an embodiment of the present invention.
FIGS. 8 to 10 are diagrams showing results of checking secondary structures of PEG-EPO-Mircera and PEG-EPO-TS according to an embodiment of the present invention.
FIG. 11 is a diagram showing results of peptide analysis after performing Lys-C degrading enzyme, chymotrypsin degrading enzyme (chymotrypsin digestion), or Glu-C degrading enzyme treatments on PEG-EPO-Mircera, PEG-EPO-TS, and EPO according to one embodiment of the present invention.
FIG. 12 is a diagram showing results of peptide analysis after performing a N-deglycosylation enzyme treatment on PEG-EPO-Mircera, PEG-EPO-TS, and EPO according to an embodiment of the present invention.
FIG. 13 is a diagram showing results of performing PEGylated EPO peptide site analysis of PEG-EPO-TS and PEG-EPO-Mircera according to an embodiment of the present invention.
FIG. 14 is a peptide schematic diagram showing PEGylated EPO peptide sites of PEG-EPO-TS and PEG-EPO-Mircera according to an embodiment of the present invention.
FIG. 15 is a diagram showing results of measuring half-maximal effective concentration (EC50) of EPO, PEG-EPO-TS, and PEG-EPO-Mircera according to an embodiment of the present invention.
FIG. 16 is a diagram showing results of measuring changes in blood concentration of EPO, PEG-EPO-TS, or PEG-EPO-Mircera over time according to an embodiment of the present invention.

### DESCRIPTION OF THE PREFERRED EMBODIMENTS

Hereinafter, the present invention is specifically described with reference to examples. These examples are only for specifically describing the present invention.

### Example 1. Preparation of PEG-EPO conjugate

The reaction was performed in a 50 mM of phosphoric acid salt (sodium phosphate, pH 7.5) solution at a ratio of EPO:PEG = 1:5. To this end, the EPO, which had been refrigerated, was stored in ice and PEG was warmed up to reach room temperature before the reaction. Five ratios of PEG was dissolved in phosphoric acid salt first by using a magnetic bar, one ratio of EPO was added, and stirring was performed for one hour at room temperature (23°C to 25°C) to be PEGylated. At this point, the final concentration of EPO was 1 mg/ml. After the stirring, pH was set to 4.4 to 4.5 by using acetic acid to stop the reaction between PEG and EPO. An hour after the reaction, the reaction yield was analyzed by HPLC, and after completion of the reaction, the final product was diluted 5-fold with 10 mM of a sodium acetate (NaOAc) buffer (pH 4.4).

The amino acid sequence of the human-derived EPO used in the present invention is set forth in SEQ ID NO: 1, the PEGylated EPO prepared by the preparation method of the present invention is named "PEG-EPO-TS", and a chemical formula of the polyethylene glycol is shown in Chemical Formula 1 below.

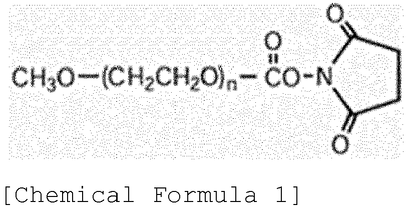

As a comparative group of the composition of the present invention, commercially available pegylated EPO (Mircera, Roche Korea Co., Ltd.) was used. This was named "PEG-EPO-Mircera".

### Example 2. Analysis on molecular weight of PEG-EPO conjugate

Protein molecular weight analysis was performed on the PEG-EPO-TS prepared in Example 1 by using a MALDI-TOF (matrix-assisted laser desorption ionization time of flight) mass spectrometer. First, 0.5 ml of a stock solution (770 ug/ml) of a sample and BSA (Calibration standard bovine serum albumin) as a control group were mixed with 10 mg/ml of sinapic acid (30% of acetonitrile and 0.3% of trifluoroacetic acid) in the ratio of 1:1 (v/v), and 1 µl was added dropwise to an OptiTOF MALDI plate (stainless steel target plate, ABSCIEX) and was dried in a vacuum centrifugal concentrator. The MALDI-TOF mass spectrometer performance conditions are shown in Table 1 below.

**[Table 1]**

| Analysis mode | MS linear high mass positive ion mode |
|---|---|
| Mass range (Da) | 10,000 to 100,000 |
| Total shots/spectrum | 2,250 shots |
| Fixed laser intensity | 7,200 |
| Detector voltage multiplier | 0.98 |

Under the above conditions, results of the BSA control group measurement were 66,425.8438 (monovalent charged ions) and 33,222.5117 (bivalent charged ions). Due to the characteristic of the linear mode which is a method of measuring a sample having a large molecular weight, average MS is used, and thus it is obvious that the accuracy of the mass is lower than that in a reflector mode. Therefore, based on the BSA control group measurements, the MS tolerance after mass correction is set to +/- 10 m/z to perform the PEG-EPO-TS sample analysis. As a result of repeatedly performing the measurement on the same PEG-EPO-TS sample six times, each measured value was counted as 59,374.58 ± 83.77 as a result of mean and standard deviation treatment, and thus it was confirmed that the PEG-EPO-TS prepared in the present invention was 59 kDa protein. The average value of the results of the six repeated measurements is shown in Table 2 below.

**[Table 2]**

| Sample | Charge (n) | (M+nH)n + Average |
|---|---|---|
| PEG-EPO-TS | +1 | 59,465.3672 |
| | +1 | 59,376.9063 |
| | +1 | 59,253.4570 |
| | +1 | 59,324.4727 |
| | +1 | 59,471.1094 |
| | +1 | 59,356.1914 |
| PEG-EPO-TS Intact MS results (Average ± STDEV): 59,374.58±83.77 | | |

### Example 3. Separation and purification of PEG-EPO conjugate

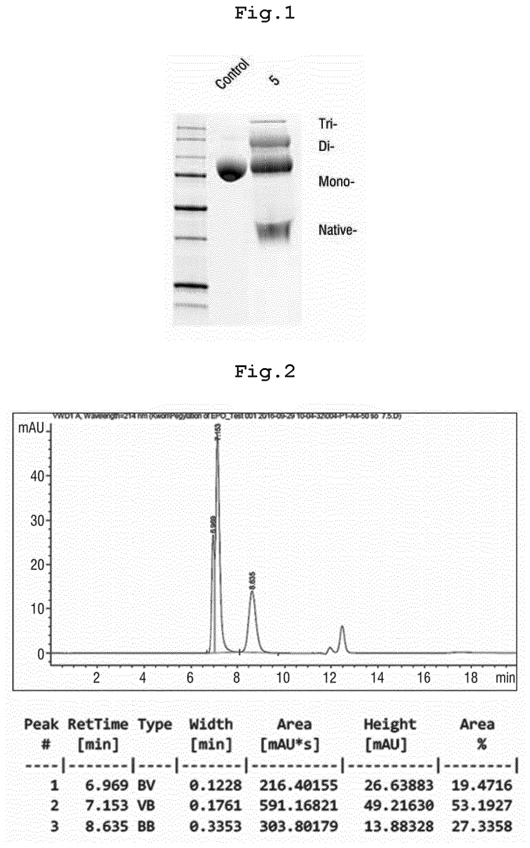

The SDS PAGE analysis of the PEG-EPO conjugate is shown in FIG. 1, and the result of performing HPLC purity analysis is shown in FIG. 2. With respect to the HPLC purity analysis, by a method of diluting and washing the PEG-EPO-TS conjugate mixture with 10 mM of sodium acetate pH 4.4 buffer or 10 mM of sodium acetate pH 4.4 buffer added with hydrochloric acid salt, separation and purification of the PEG-EPO-TS conjugates were performed by cation exchange chromatography. A resin of chromatography was measured with 30 ml of SP Sepharose Fast Flow (GE Healthcare, Cat #17-0729-01) at a flow rate of 5 ml/min. More specific chromatographic performance conditions are listed in Table 3 below.

**[Table 3]**

| Fraction | Buffer | Column volume |
|---|---|---|
| Equilibration | 10 mM NaOAc pH 4.4 buffer | 5 CV |
| Loading | Product | 100 ml |
| Washing | 10 mM NaOAc pH 4.4 buffer+50 mM NaCl | 10 CV |
| Elution 1 | 10 mM NaOAc pH 4.4 buffer+110 mM NaCl | 12 CV |
| Elution 2 | 10 mM NaOAc pH 4.4 buffer+180 mM NaCl | 12 CV |
| Elution 3 | 10 mM NaOAc pH 4.4 buffer+1 M NaCl | 10 CV |

As results of the experiment, it was found that ratios of a native, a monomer, a dimer, and a trimer of the PEG-EPO-TS conjugate were 19.47% for the dimer and the trimer, 59.19% for the monomer, and 27.3% for the native.

### Example 4. Production of PEG-EPO conjugate and checking of purification purity

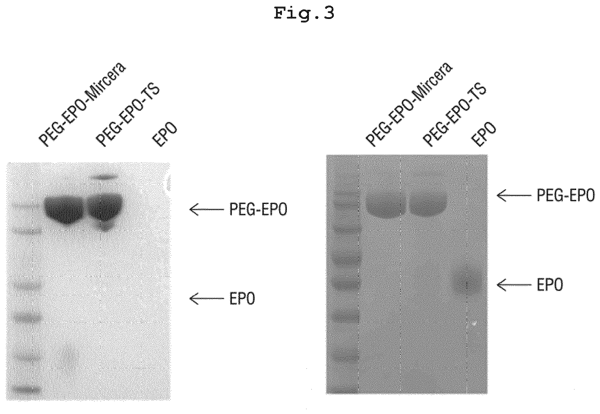

Commercially available PEG-EPO-Mircera (mircera, Roche Korea, Co., Ltd.) as a positive control group and PEG-EPO-TS and EPO of the present invention were trizol stained. Protein size fractionation was first performed on the three kinds of samples in SDS-PAGE, and the SDS-PAGE was washed for one minute in DDW at 70°C and then stained for five minutes in 25 ml of barium chloride solution (5%) at 70°C. After washing for 5 minutes in DDW, coloring was performed for one minute in 25 ml iodine solution. Once protein bands were identified, DDW washing was performed. The results immediately after the SDS-PAGE fractions and after staining are shown in FIG. 3.

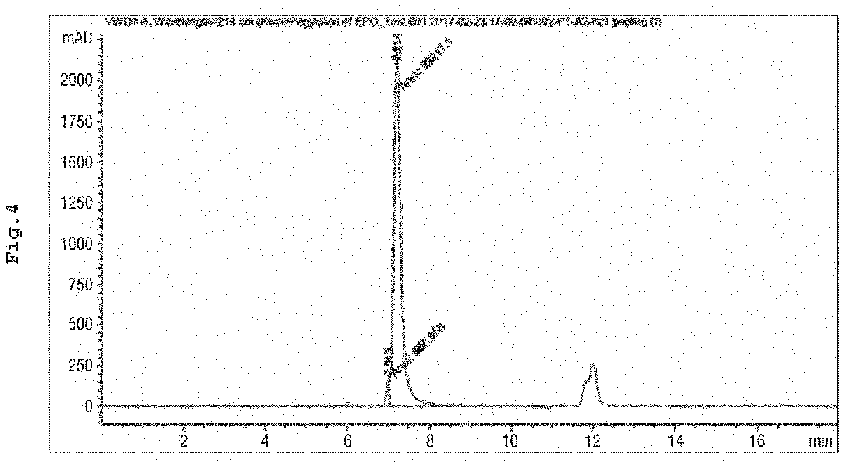

In addition, the protein was extracted from the band at the PEG-EPO-TS position in the SDS-PAGE-fractionated band and analyzed by HPLC in the same manner as in Example 3, to find that the dimer and the trimer were 2.3%, the monomer was 97.6%, and the native was 0%. Therefore, it was confirmed that the purification of the monomer was very well done. The results are shown in FIG. 4.

### Example 5. Checking of optimal conditions for preparation of PEG-EPO conjugate

From results of experiment of Examples 1 to 4, the stable production of PEG-EPO-TS was confirmed, and in order to optimize the PEGylation reaction conditions of PEG-EPO-TS, and optimization experiments depending on pH, a molar ratio, and reaction time were performed.

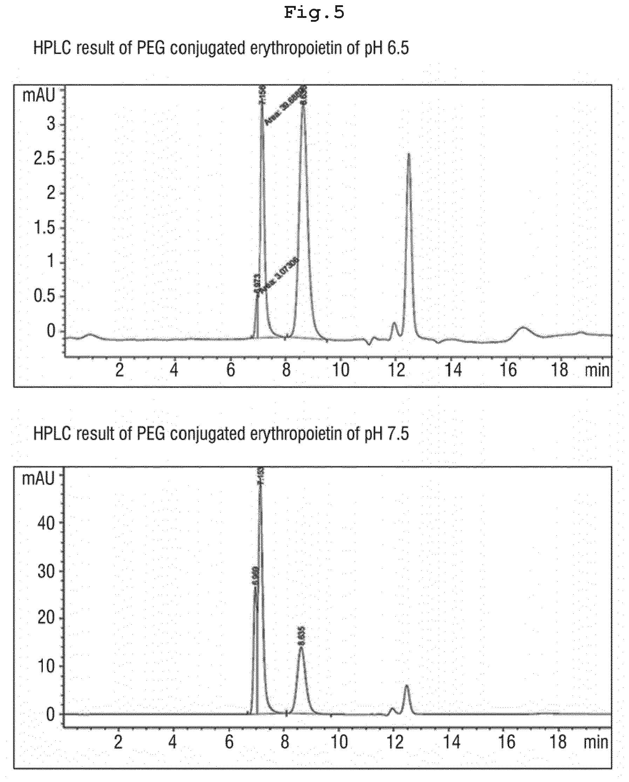

First, by HPLC analysis after PEGylation at reaction conditions of pH 6.5 or pH 7.5, production ratios of the monomer, the dimer, and the native of PEG-EPO-TS were checked. The results are shown in Table 4 and FIG. 5.

**[Table 4]**

| pH | pH 6.5 | | pH 7.5 | |
|---|---|---|---|---|
| Sample | Protein (mg) | Yield (%) | Protein (mg) | Yield (%) |
| Rxn. Mix | 20 | 100 | 20 | 100 |
| Mono- | 6.8 | 34 | 10.62 | 53.1 |
| Di- | 0.66 | 3.3 | 3.88 | 19.4 |
| Unmod | 11.12 | 55.6 | 5.46 | 27.3 |
| Conju.Mix | 7.46 | 57 | 14.5 | 76.5 |

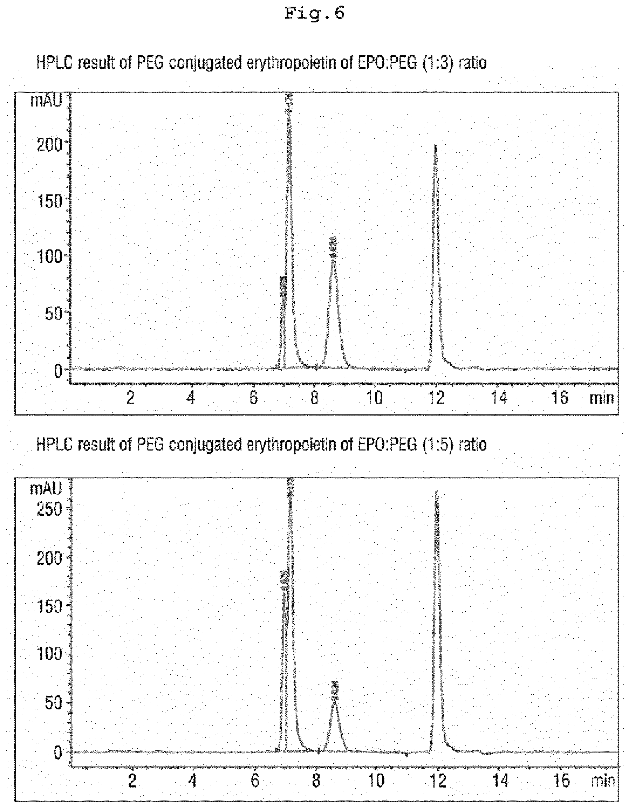

Next, in order to analyze the degree of PEGylation depending on the molar ratio, PEGylation was performed under the condition of an EPO and PEG ratio of 1:3 or 1:5 at pH 7.5, and production ratios of the monomer, the dimer, and the native of PEG-EPO-TS were checked by HPLC analysis. The results are shown in Table 5 and FIG. 6.

**[Table 5]**

| Ratio | Ratio (1:3) | | Ratio (1:5) | |
|---|---|---|---|---|
| Sample | Protein (mg) | Yield (%) | Protein (mg) | Yield (%) |
| Rxn. Mix | 20 | 100 | 20 | 100 |
| Mono- | 8.2 | 41 | 11.99 | 57.1 |
| Di- | 3.2 | 16 | 3.88 | 19.4 |
| Unmod | 8.6 | 43 | 9.58 | 23.5 |
| Conju.Mix | 11.4 | 57 | 15.3 | 76.5 |

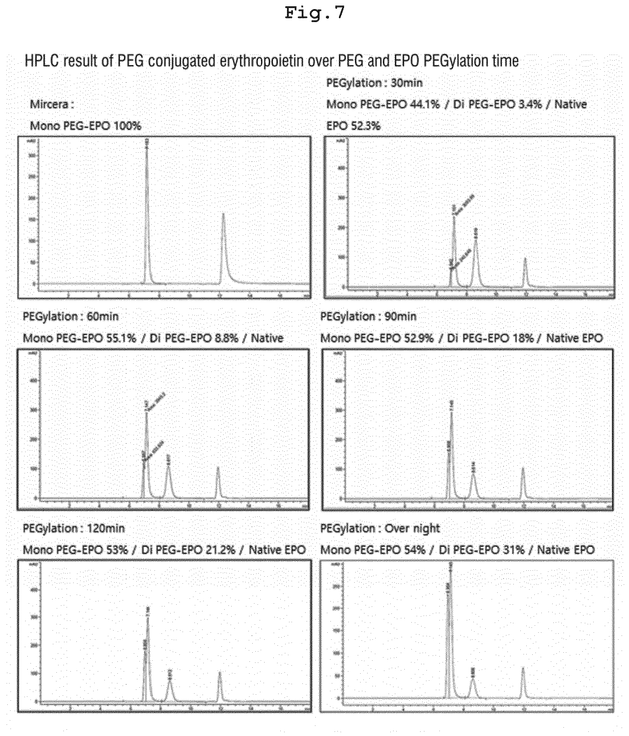

Finally, the production ratios of the monomer, the dimer, and the native of PEG-EPO-TS after PEGylation depending on the reaction time were checked under the conditions of pH 7.5 and the EPO to PEG ratio of 1:5. The results are shown in Table 6 and FIG. 7.

**Table 6**

| Time | 30 min | | 60 min | | 90 min | | 120 min | | Overnight | |
|---|---|---|---|---|---|---|---|---|---|---|
| Sample | Prot ein (mg) | Yiel d (%) | Prot ein (mg) | Yiel d (%) | Prot ein (mg) | Yiel d (%) | Prot ein (mg) | Yiel d (%) | Prot ein (mg) | Yiel d (%) |
| Rxn. Mix | 20 | 100 | 20 | 100 | 20 | 100 | 20 | 100 | 20 | 100 |
| Mono- | 8.2 | 44.1 | 8.2 | 55.1 | 10.5 8 | 52.9 | 10.6 | 53 | 10.8 | 54 |
| Di- | 3.2 | 3.4 | 3.2 | 8.8 | 3.6 | 18 | 4.24 | 21.2 | 6.2 | 31 |
| Unmod | 8.6 | 52.3 | 8.6 | 35.9 | 5.8 | 29 | 5 | 25 | 2.8 | 14 |
| Conju.M ix | 11.4 | 47.7 | 11.4 | 64.1 | 14.1 8 | 71 | 14.8 4 | 75 | 17 | 86 |

### Example 6. Confirming of secondary structure of PEG-EPO conjugate

In order to confirm the structural differences and the significance of commercially available PEG-EPO-Mircera as the positive control group and PEG-EPO-TS, protein structure analysis was performed. To this end, first, 50 mM of a sodium phosphate (pH 6 to 8) buffer was prepared, the sample was diluted to 0.2 mg/ml to 0.5 mg/ml, and 200 µl was charged in a 1-mm cell, and UV measurement was performed at 260 to 180 nm (Far UV J-1500, JASCO International Co., Ltd). Secondary structure analysis of proteins was performed with the program of JASCO Spectra Manager Version 2 CD Multivarlate SSE version 2.1.0.3.

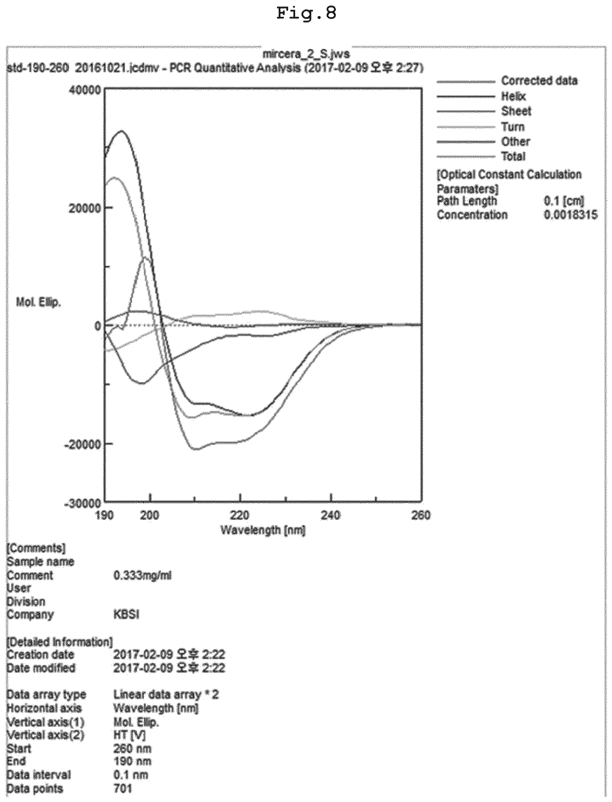

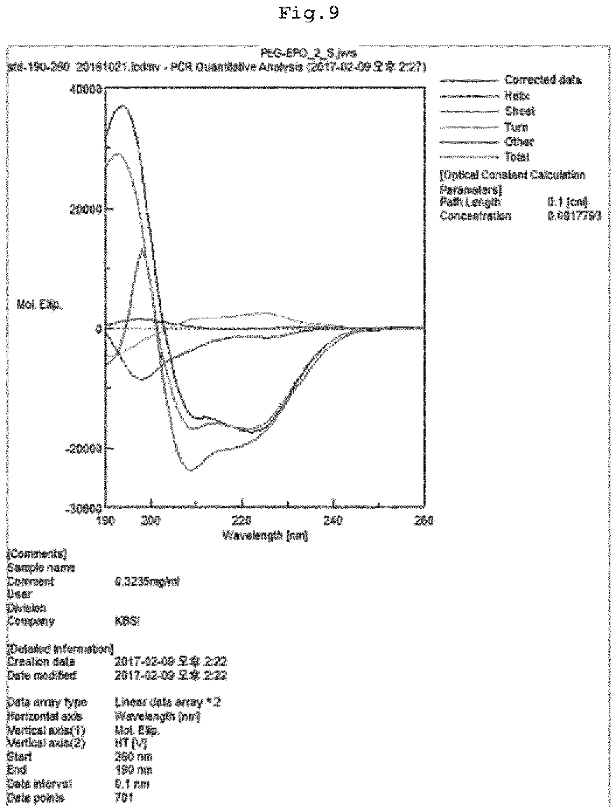

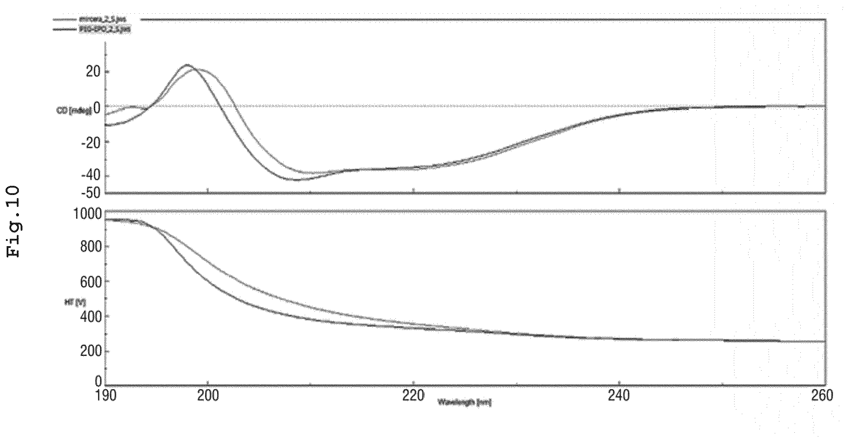

As results of the experiment, it was checked that there was a great difference, particularly in the 200 to 210 nm section. The results are shown in Table 7 and FIGS. 8 to 10.

**[Table 7]**

| Sample | Helix | Sheet | Turn | Other |
|---|---|---|---|---|
| PEG-EPO-Mircera | 49.90% | 9.00% | 9.60% | 31.50% |
| PEG-EPO-TS | 56.60% | 5.70% | 10.50% | 27.20% |

### Example 7. Confirming of conjugation location and peptide sequence of PEG-EPO conjugate

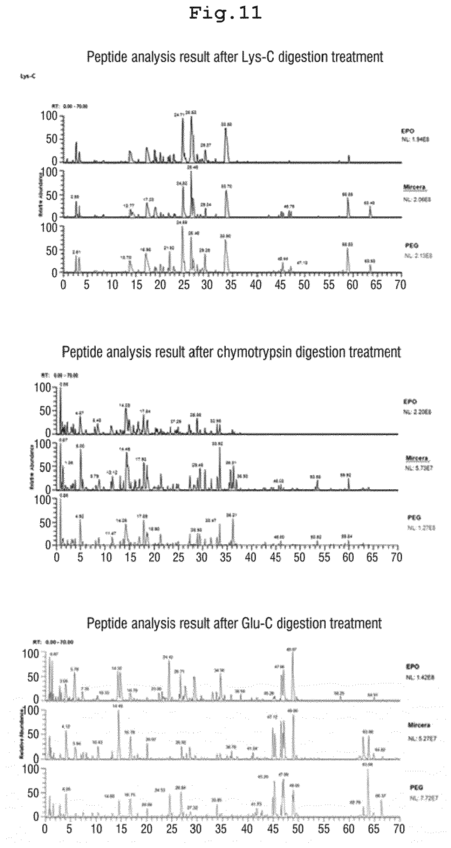

In order to check the conjugation position of the PEG-EPO-TS prepared in the present invention, peptide sequence analysis was performed with EPO protein as a negative control group and commercially available PEG-EPO-Mircera as a positive control group. The signal protein sequence of EPO for PEGylation is set forth in SEQ ID NO: 2. The peak patterns of peptide mapping after performing Lys-C degrading enzyme, chymotrypsin degrading enzyme (chymotrypsin digestion), or Glu-C degrading enzyme treatments were analyzed. As results of the experiment, it was confirmed that, even after three kinds of enzyme treatments, peak patterns of EPO protein, PEG-EPO-TS, and PEG-EPO-Mircera were evenly appeared. The results are shown in FIG. 11.

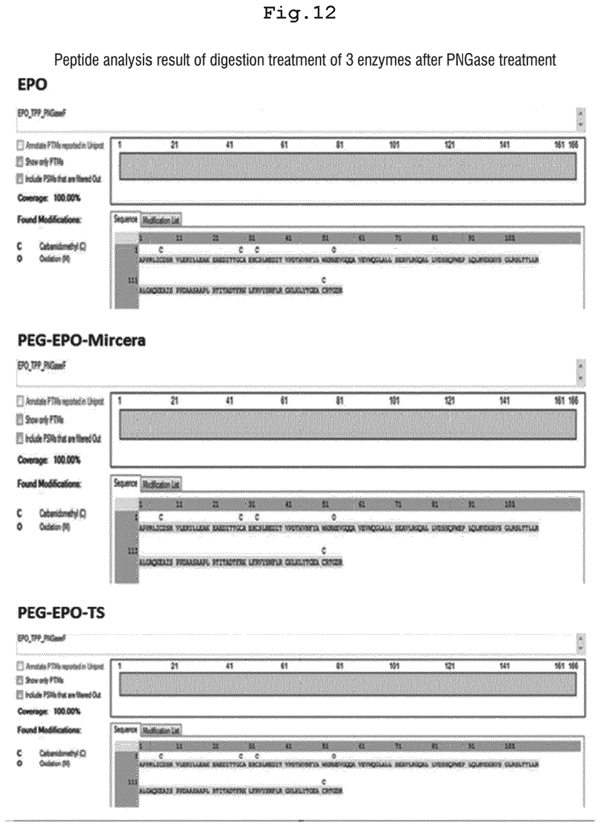

In addition, after a peptide N-deglycosylase (PNGase) treatment was performed on EPO protein, PEG-EPO-TS, or PEG-EPO-Mircera, peptide sequence analysis was performed on the three enzymes-treated samples. As a result of the comparison by using a mirror image, it was confirmed that the retention time for each peak showed the same pattern according to the molecular weight. However, there was a slight difference between PEG-EPO-TS and PEG-EPO-Mircera, and it was predicted that this difference is seen in three N-glycans and one O-glycan when PNGase F was not treated due to the characteristics of EPO. The results are shown in FIG. 12.

Finally, PEGylation site analysis was performed on the three enzyme-treated PEG-EPO-TS and PEG-EPO-Mircera. First, 0.5 mg of the protein sample was diluted with 25 ul of ammonium bicarbonate (100 mM), then 25 ul of a TFE denatured solution was added, 1 µl of DTT (200 mM) was mixed, and the resultant was heated at 60°C for one hour and cooled. Thereafter, 4 µl of IAM (200 mM) was added and incubated for one hour in the dark at room temperature, 1 µl of DTT (200 mM) was added in order to stop the reaction of IAM, and incubation was further performed for one hour in the dark at room temperature. 300 µl of distilled water was added to a tube including the reaction solution, and100 µl of ammonium bicarbonate (100 mM) was added in order to increase pH. After it was checked that the pH was 7.5 to 8.0, trypsin was added in a 1:50 ratio, incubation was performed overnight at 37°C, and 1 µl of formic acid or TFA was further added. Determination of a protein degradation form was performed by Acquity UPLC I-Class equipment, and an Advance biopeptide mapping column (2.1 X 150 mm, Agilent) was used as a column. The temperature in use was set to 60°C, and the flow rate was adjusted to 0.4 ml per minute. The linear gradient was adjusted from 5% to 40% per one hour. The measurement was performed by using Orbitrap Elite instrument as a mass spectrometer for mass spectrometry and by setting the resolution to 60,000, the mode to a cation, Max IT to 100 ms, the scan range to 400 to 2,000, the active type to CID, a minimum required signal to 5.00E+01, an isolation width to 5.0 m/s, NCE to 35, the activation time to 30 ms, and the spectrum to Centroid.

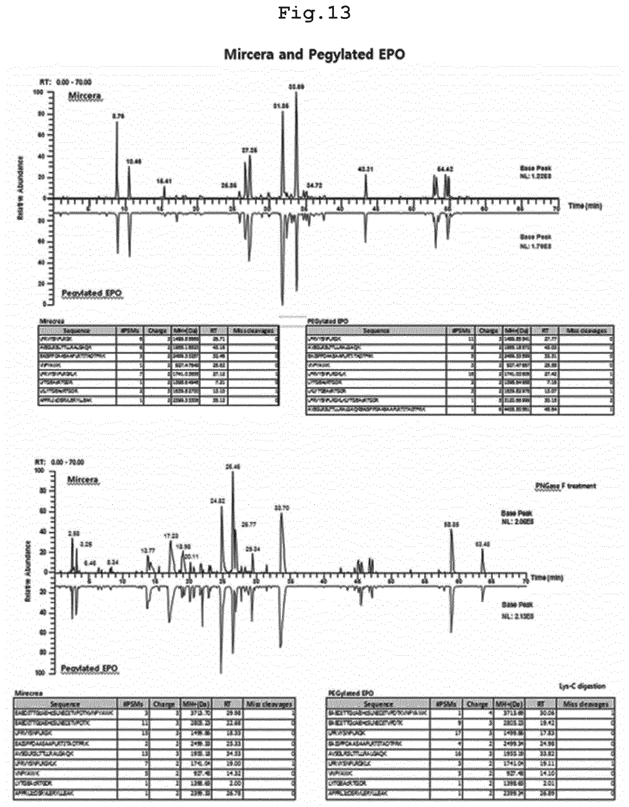

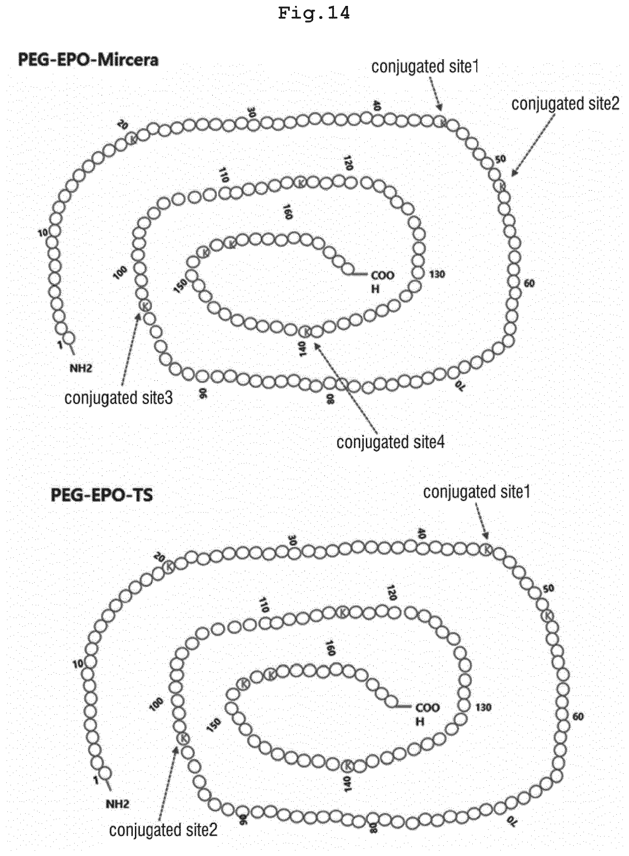

As results of the experiment, it was found that, PEG-EPO-TS and PEG-EPO-Mircera had different sites of PEGylated EPO peptides. It was confirmed that PEG-EPO-TS prepared in the present invention was PEGylated at Lys45 and Lys97 amino acids, but PEG-EPO-Mircera was PEGylated at Lys45, Lys52, Lys97, and Lys140 amino acids. The results are shown in FIG. 13, and a peptide schematic diagram indicating the PEGylation sites is shown in FIG. 14.

### Example 8. Confirming of half-maximal effective concentration (EC50) of PEG-EPO conjugate

Half-maximal effective concentrations (EC50) of EPO, PEG-EPO-TS, and PEG-EPO-Mircera were measured. Measurements were performed according to the recommended protocol of CellTiter-Blue Cell viability assay (Promega, Cat. G8080) kit manufacturer with reference to the paper of Nett JH, et al. (J Biotechnol. 2012 Jan; 157(1): 198-206).

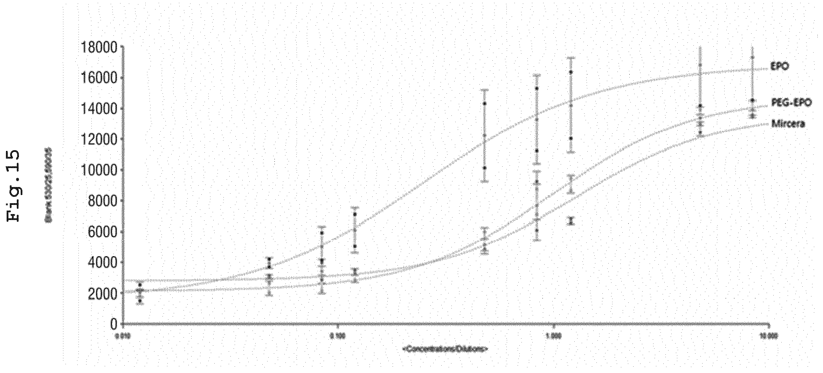

Specifically, one day before the experiment, TF-1 cells (human erythroblast, ATCC, Cat. RL-2003) were obtained from a T75 culture dish and centrifuged at 125 g for seven minutes, and 10 ml of 1X PBS was added to the pellet from which the supernatant was removed to wash the cells. The resultant was further centrifuged at 125 g for seven minutes to remove the 1X PBS in the same manner as above and diluted with 5 ml Opti-MEM (Gibco, Cat.3198-070) culture medium containing 2% FBS to perform counting, re-dilution to 3 × 10⁵ cells/ml was performed to perform dispensation, and O/N incubation was performed. On the following day, a pre-treatment is performed by diluting the EPO, PEG-EPO-TS, and PEG-EPO-Mircera conjugates with a serum-free Opti-MEM culture medium on microtubes to be 100 ng/ml, the conjugates were serially diluted down consecutively nine times by 1/2, samples were prepared to allow duplicate analysis for each concentration, and was added in 30 µl per well to a 96 well plate in which 20 µl of the Opti-MEM culture medium was dispensed. Thereafter, cultured cells were obtained, counted, and added to the 96 well plates by 1 × 10⁴ cells/50 µl per well. As the control group, wells only containing the Opti-MEM culture media or the cells were prepared together. The wells to which the cells were added were well shaken to disperse the cells and re-cultured for three days (72 hours) in a 37°C incubator. After 3 days, 20 µl of a CellTiter-Blue Cell reagent was added to each well of the plate, reacted for four hours, and then the absorbance was measured. The measurement mode of the spectrophotometer (Microplate reader, BioTek, Synerge HT) was set to fluorescence, the lead type was set to the endpoint, and the wavelength was set to 560 ex/590 em. Results of testing above measurement results by a parallel line test and calculating the titer of the sample are provided in FIG. 15.

As results of the experiment, it was found that half-maximal effective concentrations (EC50) were 0.252 for EPO, 0.994 for PEG-EPO-TS, and 1.269 for PEG-EPO-Mircera, and thus it was understood that PEG-EPO-TS had a half-maximal effective concentration lower than that of PEG-EPO-Mircera.

### Example 9. Confirming of pharmacokinetic effects of PEG-EPO conjugate

### Example 9-1. Confirming of pharmacokinetics in rat

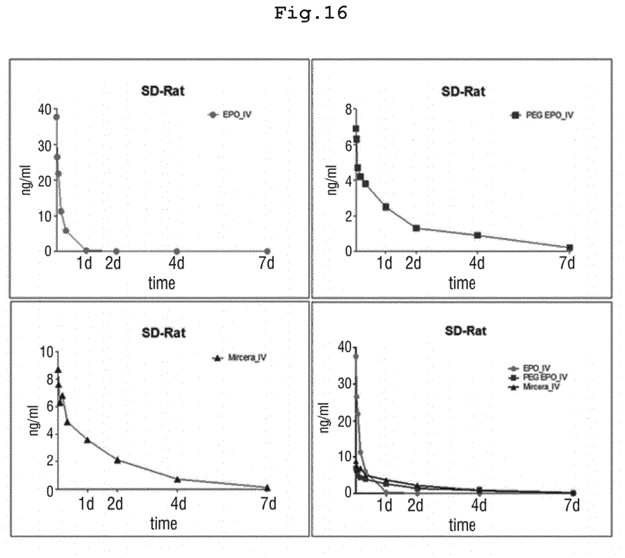

Pharmacokinetics were measured by a method of intravenously administrating PEG-EPO conjugates to SD rats (male, 7 weeks old, body weight of 243.53±14.33 g). Specifically, after the administration of 3 µg/kg of EPO, PEG-EPO-TS, or PEG-EPO-Mircera, after 0, 0.5, 1, 2, 4, 8, 24 (1 d), 48 (2 d), 96 (4 d), and 168 (8 d) hours, blood was collected from the rats with an SST tube, centrifuged at 6,000 rpm for 10 minutes at 4°C, and stored at -80°C until the concentration measurement. The concentration of erythropoietin in the blood was measured by ELISA. The changes in concentrations of the drugs in the blood over time are shown in Table 8 and FIG. 16.

**[Table 8]**

| | 0 h | 0.5 h | 1h | 2h | 4h | 8h | 24h | 48h | 96h | 168h |
|---|---|---|---|---|---|---|---|---|---|---|
| EPO | 0 | 37.6 | 26.5 | 21.8 | 11.3 | 5.9 | 0.3 | 0 | 0 | 0 |
| PEG-EPO-TS | 0 | 6.9 | 6.3 | 4.7 | 4.2 | 3.8 | 2.5 | 1.3 | 0.9 | 0.2 |
| PEG-EPO-Mircera | 0 | 8.7 | 7.6 | 6.3 | 6.8 | 4.9 | 3.6 | 2.1 | 0.7 | 0.1 |

The pharmacokinetic analysis of the measured concentration results was performed by non-compartmental analysis (NCA), and the analysis was performed with the program of Phoenix WinNonlin 7.0 (Certara USA Inc., Princeton, NJ, USA). Individual NCA parameters were calculated. The results are shown in Table 9 below.

**[Table 9]**

| | EPO | PEG-EPO-TS | PEG-EPO-Mircera |
|---|---|---|---|
| Body weight (g) | 264.7 | 238.1 | 257.1 |
| T_{1/2} (h) | 3.79 | 38.21 | 27.83 |
| Tₘₐₓ (h) | 0.5 | 0.5 | 0.5 |
| Cₘₐₓ (ng/ml) | 37.6 | 6.9 | 8.7 |
| C₀ (ng/ml) | 53.35 | 7.56 | 9.96 |
| AUCₗₐₛₜ (ng·h/ml) | 180.01 | 225.71 | 284.59 |
| AUC_{inf} (ng·h/ml) | 181.65 | 236.74 | 288.60 |
| AUC_{%Extrap} (%) | 0.90 | 4.66 | 1.39 |
| V_{Z} (ml/kg) | 903.47 | 6469.54 | 4506.67 |
| C₁ (ml/h/kg) | 165.15 | 117.36 | 112.24 |
| MRTₗₐₛₜ (h) | 4.22 | 45.26 | 36.87 |
| Vₛₛ (ml/kg) | 734.81 | 6283.26 | 4406.32 |
| T_{1/2}, half-life; Tₘₐₓ, time at maximal concentration; Cₘₐₓ, maximal concentration; C₀, initial concentration; AUCₗₐₛₜ, area under the curve from administration to the last measured concentration; AUC_{inf}, area under the curve from administration to infinity; AUC_{%Extrap}, percentage of the extrapolated area under the curve at the total area under the curve; V_{Z}, volume of distribution; C₁, clearance; MRTₗₐₛₜ, mean residence time to the last measured concentration; Vₛₛ, volume of distribution at steady state | | | |

As a result of the experiment, in case of IV administration, the half-life was 3.8 h for EPO, 38.2 h for PEG-EPO-TS, and 27.8 h for PEG-EPO-Mircera, and it was found that the half-life of PEG-EPO was 7 to 12 times longer than that of EPO. Also, in the comparison between PEG-EPO conjugates, it was found that PEG-EPO-TS had the 27% longer half-life than PEG-EPO-Mircera.

### Example 9-2. Confirming of pharmacodynamics in mice

Pharmacodynamics were measured by a method of intraperitoneally administrating the PEG-EPO conjugates to B6D2F1 mice (female, 7 weeks old, body weight of 20.02±0.88 g). Specifically, 1.25, 2.5, 5, or 20 µg/kg of EPO, PEG-EPO-TS, or PEG-EPO-Mircera drugs were administered, after 7 days, 200 to 300 µl of the blood was collected from the facial vein by using a lancet into a micro EDTA tube (MiniCollect, Greiner Bio One International GmbH), the shaker was used to sufficiently mix the EDTA to prevent coagulation, and then, the CBC test was performed by using the ADVIA 2120i (Siemens AG) equipment within one hour. The results are shown in Table 10 below with the comparison of CBC results before drug administration.

**[Table 10]**

| | Dose(µg/ kg) | RBC(×10⁶/µl) | | | Reticuloc yte (%) | | Reticulocyte (×10⁹/L) | | |
|---|---|---|---|---|---|---|---|---|---|
| | | 0d | 7d | Increa se (%) | 0d | 7d | 0d | 7d | Increa se (%) |
| Vehic le | 0 | 10. 84 | 10. 21 | -5.81 | 1.6 0 | 5.78 | 173. 20 | 589.8 0 | 240.53 |
| EPO | 1.25 | 10. 52 | 10. 05 | -4.47 | 2.3 0 | 4.87 | 242. 20 | 489.5 0 | 102.11 |
| | 2.5 | 9.8 7 | 10. 45 | 5.88 | 1.9 6 | 4.93 | 193. 70 | 515.1 0 | 165.93 |
| | 5. | 10. 20 | 10. 52 | 3.14 | 2.0 4 | 3.51 | 208. 30 | 368.9 0 | 77.10 |
| | 20 | 10. 77 | 11. 19 | 3.90 | 1.5 3 | 1.80 | 165. 30 | 200.9 0 | 21.54 |
| PEG-EPO-TS | 1.25 | 10. 50 | 11. 09 | 5.62 | 1.1 8 | 2.50 | 123. 60 | 277.2 0 | 124.27 |
| | 2.5 | 10. 59 | 12. 25 | 15.68 | 1.6 1 | 6.38 | 170. 30 | 781.0 0 | 358.60 |
| | 5. | 9.4 7 | 13. 04 | 37.70 | 1.7 3 | 14.2 3 | 163. 70 | 1855. 00 | 1033.1 7 |
| | 20 | 10. 47 | 14. 10 | 34.67 | 0.8 5 | 15.7 8 | 89.2 0 | 2225. 00 | 2394.3 9 |
| PEG-EPO-Mirce ra | 1.25 | 8.2 5 | 9.4 9 | 15.03 | 2.7 7 | 4.78 | 228. 40 | 453.4 0 | 98.51 |
| | 2.5 | 8.8 6 | 12. 16 | 37.25 | 1.6 2 | 4.05 | 143. 50 | 491.9 0 | 242.79 |
| | 5. | 9.1 5 | 12. 01 | 31.26 | 1.4 7 | 12.8 9 | 134. 10 | 1548. 00 | 1054.3 6 |
| | 20 | 10. 43 | 13. 55 | 29.91 | 1.8 7 | 17.3 3 | 194. 70 | 2347. 00 | 1105.4 4 |

As a result of the experiment, in case of erythrocyte (RBC), the vehicle administration group showed a 5.81% of decrease (-5.81%), the EPO administration group showed a decrease or increase of -4.47% to 5.88%, but it was confirmed that the PEG-EPO-TS administration group showed an increase of 5.62% to 37.70%. This shows a prominent increase of the number of erythrocytes than PEG-EPO-Mircera which showed an increase of 3.3% to 37.24%. In case of reticulocytes, it was found that the increase amount of the number of reticulocytes in the EPO administration group was smaller than that in the vehicle administration group (vehicle vs. EPO = 240% vs. 21.54 to 165.93%). In the PEG-EPO-Mircera administration group, the increase amount was 98.51% to 1,105.44%, and it was found that the increase amount was relatively large and well reflected the increase depending on concentration. In case of the PEG-EPO-TS administration group, the increase amount was 124.27% to 2,394.39%, and it was found that, among the three groups, the increase amount was the largest and well reflected the increase depending on concentration.

### INDUSTRIAL APPLICABILITY

The erythropoietin composition with improved stability according to the present invention increases the efficacies of the drug in the body by reducing the decrease in physiological activities of erythropoietin as much as possible and increasing blood serum half-life and can be usefully used in the treatment of diseases relating to the production of erythrocyte and hematopoiesis.

## Claims

1. A method of preparing a conjugate in which a compound represented by the formula (1) is conjugated to Lys 45 and 97 amino acids of erythropoietin, the method comprising:
(a) a step of dissolving the compound represented by the formula (1) in phosphoric acid salt;
(b) a step of adding erythropoietin in a molar ratio of 1:1 to 1:5 with respect to the compound represented by the formula (1) to (a) and stirring at 18° C to 32° C for 0.2 to 3 hours; and
(c) a step of stopping reaction by adjusting acidity (pH) to 4.0 to 5.0

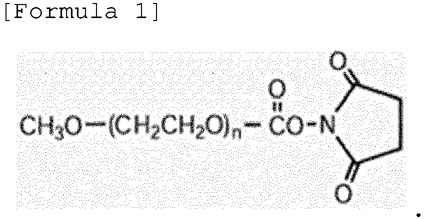

2. A conjugate obtainable by the method according to claim 1.

3. A pharmaceutical composition for use in preventing or treating a hematopoietic disease, wherein the pharmaceutical composition comprises the conjugate according to claim 2, as an active ingredient.

4. The pharmaceutical composition for use according to claim 3,
wherein the hematopoietic disease is any one or more selected from the group consisting of iron deficiency anemia, pernicious anemia, aplastic anemia, hemolytic anemia, lymphoid leukemia, myeloid leukemia, multiple myeloma, myeloid fibrosis, idiopathic thrombocytopenic purpura, thrombotic thrombocytopenia, hemophilia, a von Willebrand disease, a disseminated intravascular coagulation syndrome, nonspecific lymphadenitis, tuberculous lymphadenitis & sarcoidosis, necrotizing lymphadenitis, lymphoid malignancy, spleen disease, and thymic disease.

## Patentansprüche

1. Verfahren zur Herstellung eines Konjugats, in dem eine Verbindung, dargestellt durch die Formel (1), an Aminosäuren Lys 45 und 97 von Erythropoietin konjugiert ist, wobei das Verfahren umfasst:
(a) einen Schritt des Lösens der Verbindung, dargestellt durch die Formel (1), in Phosphorsäuresalz;
(b) einen Schritt der Zugabe von Erythropoietin in einem Molverhältnis von 1:1 bis 1:5 in Bezug auf die Verbindung, dargestellt durch die Formel (1), zu (a) und Rühren bei 18°C bis 32°C für 0,2 bis 3 Stunden; und
(c) einen Schritt des Stoppens der Reaktion durch Einstellen der Azidität (pH) auf 4,0 bis 5,0

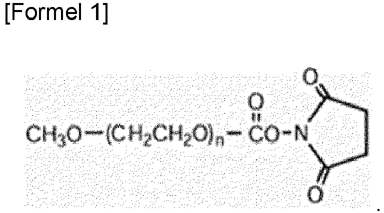

2. Konjugat, erhältlich mit dem Verfahren nach Anspruch 1.

3. Pharmazeutische Zusammensetzung zur Verwendung bei der Prävention oder Behandlung einer hämatopoetischen Erkrankung, wobei die pharmazeutische Zusammensetzung das Konjugat nach Anspruch 2 als einen Wirkstoff umfasst.

4. Pharmazeutische Zusammensetzung zur Verwendung gemäß Anspruch 3,
wobei die hämatopoetische Erkrankung eine oder mehrere ist, ausgewählt aus der Gruppe, bestehend aus Eisenmangelanämie, perniziöser Anämie, aplastischer Anämie, hämolytischer Anämie, lymphoider Leukämie, myeloischer Leukämie, multiplem Myelom, myeloischer Fibrose, idiopathischer thrombozytopenischer Purpura, thrombotischer Thrombozytopenie, Hämophilie, einer von-Willebrand-Erkrankung, einem disseminierten intravaskulären Koagulationssyndrom, nicht-spezifischer Lymphadenitis, tuberkulöser Lymphadenitis & Sarkoidose, nekrotisierender Lymphadenitis, lymphoider Malignität, Milzerkrankung und Thymuserkrankung.

## Revendications

1. Procédé de préparation d'un conjugué dans lequel un composé représenté par la formule (1) est conjugué aux acides aminés Lys 45 et 97 de l'érythropoïétine, le procédé comprenant :
(a) une étape de dissolution du composé représenté par la formule (1) dans un sel d'acide phosphorique ;
(b) une étape d'ajout d'érythropoïétine dans un rapport molaire de 1:1 à 1:5 par rapport au composé représenté par la formule (1) à (a) et d'agitation entre 18 °C et 32 °C pendant 0,2 à 3 heures ; et
(c) une étape d'arrêt de la réaction par ajustement de l'acidité (pH) entre 4,0 et 5,0

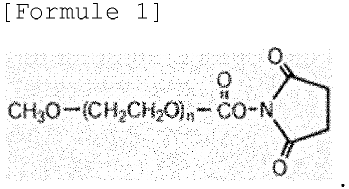

2. Conjugué pouvant être obtenu par le procédé selon la revendication 1.

3. Composition pharmaceutique destinée à être utilisée pour prévenir ou traiter une maladie hématopoïétique, dans laquelle la composition pharmaceutique comprend le conjugué selon la revendication 2, en tant qu'ingrédient actif.

4. Composition pharmaceutique destinée à être utilisée selon la revendication 3,
dans laquelle la maladie hématopoïétique est une ou plusieurs maladies choisies dans le groupe constitué d'une anémie ferriprive, une anémie pernicieuse, une anémie aplasique, une anémie hémolytique, une leucémie lymphoïde, une leucémie myéloïde, un myélome multiple, une fibrose myéloïde, un purpura thrombocytopénique idiopathique, une thrombocytopénie thrombotique, l'hémophilie, une maladie de von Willebrand, un syndrome de coagulation intravasculaire disséminée, une lymphadénite non spécifique, une lymphadénite tuberculeuse & sarcoïdose, une lymphadénite nécrosante, une tumeur maligne lymphoïde, une maladie de la rate, et une maladie thymique.
